# EUROPEAN PATENT APPLICATION

(11) **EP 3 266 379 A1**
(43) Date of publication of application: **10.01.2018**
(21) Application number: 15884007.4
(22) Date of filing: 23.10.2015
(51) Int. Cl.: A61B 8/12

(54) **SUCTION POWER ADJUSTING DEVICE FOR ULTRASOUND OBSERVATION AND ULTRASOUND ENDOSCOPE**

(30) Priority: 04.03.2015 JP 2015042378
(71) Applicant: Olympus Corporation, Tokyo 192-8507 (JP)
(72) Inventor: TSURUTA, Teppei, Tokyo 192-8507 (JP)
(74) Representative: Gunzelmann, Rainer
(86) International application number: PCT/JP2015/079968
(87) International publication number: WO 2016/139841

(57) **Abstract**

A suction force adjustment apparatus for ultrasound examination according to the invention is a suction force adjustment apparatus used for ultrasound examination of an observation target by transmitting ultrasound to the observation target and receiving the ultrasound reflected from the observation target. The apparatus includes: a suction force change unit configured such that one end is configured to be connected to a suction pump and the other end is provided at a portion of a passage leading to a contact portion with the observation target, and configured to change suction force of the suction pump on the other end; and a damper unit configured to suppress a change in the suction force by the suction force change unit.

## Description

### Field

The present invention relates to a suction force adjustment apparatus for ultrasound examination and relates to an ultrasound endoscope, used for observation of tissues as an observation target using ultrasound.

### Background

Ultrasound is applied in some case for observing characteristics of a living tissue or material as an observation target. Specifically, ultrasound transmitted toward the observation target is reflected as an ultrasound echo from the observation target, and signal processing is performed on the reflected ultrasound echo, whereby information related to the observation target is obtained.

In recent years, as an exemplary technique to obtain characteristics of an observation target using ultrasound, elastography that displays stiffness of a living tissue has been brought into practice (for example, refer to Patent Literature 1). With the elastography technology, for example, a pressed state of an ultrasound probe toward an ultrasound-detectable organ is changed into a plurality of different states, whereby a change (displacement) of the deformation state of the living tissue is measured. Subsequently, spatial differentiation is performed on this change so as to detect strain, from which an elastographic image is formed. Applying this elastography technique to an ultrasound endoscope having an ultrasound transducer on a distal end of an insertion unit, for example, would enhance the detection rate for lesions in deep organs.

### Citation List

### Patent Literature

Patent Literature 1: JP 2012-81295 A

### Summary

### Technical Problem

In order to detect a lesion (e.g. stiffness) by the elastography technique, it would be necessary to obtain ultrasound echoes having different pressed states by periodically pressing an ultrasound transducer toward a living tissue. Exemplary methods for periodically pressing the ultrasound transducer inside a subject include a method using heartbeat. In examination using heartbeat, however, the change in the pressed state is very small depending on an examined site, and this might make it difficult to stably obtain a good elastographic image.

Other exemplary methods for periodically pressing the ultrasound transducer inside the subject include a method of manually moving an insertion unit of an ultrasound endoscope forward/backward by an operator. Unfortunately, however, since a typical insertion unit of an ultrasound endoscope has flexibility and an elongated shape, forward/backward movement of the insertion unit would be likely to cause most of the amount of movement to be absorbed by deflection that occurs in the middle. Therefore, it might be difficult, also in this case, to stably obtain a good elastographic image.

As an alternative to these methods, one idea would be to provide a pressing mechanism to mechanically press the ultrasound transducer inside the subject, on a distal end portion of the ultrasound endoscope. Providing such a mechanism on the distal end portion, however, might increase the size of the distal end portion and make the structure complicated, and thus, application of this idea to the ultrasound endoscope would be undesirable.

The present invention has been made in view of the foregoing, and an object of the invention is to provide a suction force adjustment apparatus for ultrasound examination, and an ultrasound endoscope that are capable of stably obtaining a good elastographic image without increasing the size of the distal end portion, with a simple configuration.

### Solution to Problem

In order to solve the above described problem and achieve the object, a suction force adjustment apparatus according to the invention is used for ultrasound examination of an observation target by transmitting ultrasound to the observation target and receiving the ultrasound reflected from the observation target. The apparatus includes: a suction force change unit configured such that one end is configured to be connected to a suction pump and the other end is provided at a portion of a channel leading to a contact portion with the observation target, and configured to change suction force of the suction pump on the other end; and a damper unit configured to suppress a change in the suction force by the suction force change unit.

In the suction force adjustment apparatus for ultrasound examination according to the above-described invention, the suction force change unit includes: a cylinder unit having a cylindrical shape bottomed on one side, and having a first communicating portion for communicating with one end side of the channel and a second communicating portion for communicating with the other end side of the channel; a piston unit slidable with respect to the cylinder unit, and configured to change a communication state between the first communicating portion and the second communicating portion according to a movement with respect to the cylinder unit; and a spring unit configured to bias the piston unit toward a direction of causing the first communicating portion and the second communicating portion not to communicate with each other.

In the suction force adjustment apparatus for ultrasound examination according to the above-described invention, the damper unit is provided in the piston unit, and is a communication hole that allows communication between outside and a hollow portion formed by the cylinder unit and the piston unit.

In the suction force adjustment apparatus for ultrasound examination according to the above-described invention, the damper unit is an elastic member provided in a hollow portion formed by the cylinder unit and the piston unit.

The suction force adjustment apparatus for ultrasound examination according to the above-described invention further incldues an adjustment member configured to adjust volume of a hollow space formed by the communication hole.

In the suction force adjustment apparatus for ultrasound examination according to the above-described invention, the suction force change unit includes: a cylinder unit having a hollow cylindrical shape, and having a first opening portion disposed on one end to communicate with outside and a second opening portion disposed on the other end to communicate with the channel; a piston unit provided in a hollow portion of the cylinder unit and slidable between the one end and the other end of the cylinder unit; and a spring unit configured to bias the piston unit from the other end of the cylinder unit toward the one end of the cylinder unit. The damper unit is a bypass tube passage provided in the cylinder unit to allow communication between the one end and the other end of the cylinder unit. The piston unit is configured to slide with respect to the cylinder unit in accordance with one of pressure change due to the suction force and biasing force of the spring unit, thereby to open or close one end side of the bypass tube passage.

The suction force adjustment apparatus for ultrasound examination according to the above-described invention further includes an adjustment member configured to adjust a movement range of the piston unit with respect to the cylinder unit.

An ultrasound endoscope according to the invention includes: an insertion unit; an ultrasound transducer provided at a distal end of the insertion unit and configured to transmit ultrasound to an observation target and to receive the ultrasound reflected from the observation target; a suction force change unit configured such that one end is configured to be connected to a suction pump and the other end is provided at a portion of a channel leading to the distal end of the insertion unit, and configured to change suction force of the suction pump on the other end; and a damper unit configured to suppress a change in the suction force by the suction force change unit.

An ultrasound endoscope according to the invention includes: an insertion unit; an ultrasound transducer provided at a distal end of the insertion unit and configured to transmit ultrasound to an observation target and to receive the ultrasound reflected from the observation target; and a suction force adjustment unit configured such that one end leads to a suction pump and the other end is connected to a portion of a channel leading to the distal end of the insertion unit, and configured to adjust suction force of the suction pump under control of an external control apparatus. The suction force adjustment unit includes a damper unit configured to adjust a discharge of gas from the channel. Advantageous Effects of Invention

According to the present invention, it is possible to stably obtain a good elastographic image without increasing the size of the distal end portion, with a simple configuration.

### Brief Description of Drawings

FIG. 1 is a schematic diagram illustrating an ultrasound diagnosis system according to a first embodiment of the present invention.
FIG. 2 is a schematic diagram illustrating a configuration of a suction button included in an ultrasound endoscope in the ultrasound diagnosis system according to the first embodiment of the present invention.
FIG. 3 is a schematic diagram illustrating a configuration of the suction button included in the ultrasound endoscope in the ultrasound diagnosis system according to the first embodiment of the present invention.
FIG. 4 is a schematic diagram illustrating a configuration of the suction button included in the ultrasound endoscope in the ultrasound diagnosis system according to the first embodiment of the present invention.
FIG. 5 is a schematic diagram illustrating a configuration of a suction button included in an ultrasound endoscope in an ultrasound diagnosis system according to a modification of the first embodiment of the present invention.
FIG. 6 is a schematic diagram illustrating a configuration of the suction button included in the ultrasound endoscope in the ultrasound diagnosis system according to the modification of the first embodiment of the present invention.
FIG. 7 is a schematic diagram illustrating a configuration of a suction button included in an ultrasound endoscope in an ultrasound diagnosis system according to a second embodiment of the present invention.
FIG. 8 is a schematic diagram illustrating a configuration of a suction button included in an ultrasound endoscope in an ultrasound diagnosis system according to a third embodiment of the present invention.
FIG. 9 is a schematic diagram illustrating a configuration of the suction button included in the ultrasound endoscope in the ultrasound diagnosis system according to the third embodiment of the present invention.
FIG. 10 is a schematic diagram illustrating a configuration of a suction button included in an ultrasound endoscope in an ultrasound diagnosis system according to a fourth embodiment of the present invention.
FIG. 11 is a schematic diagram illustrating a configuration of a suction force adjustment unit included in an ultrasound endoscope in an ultrasound diagnosis system according to a fifth embodiment of the present invention.

### Description of Embodiments

Hereinafter, modes for carrying out the present invention (hereinafter, referred to as embodiment(s)) will be described with reference to the attached drawings.

### (First Embodiment)

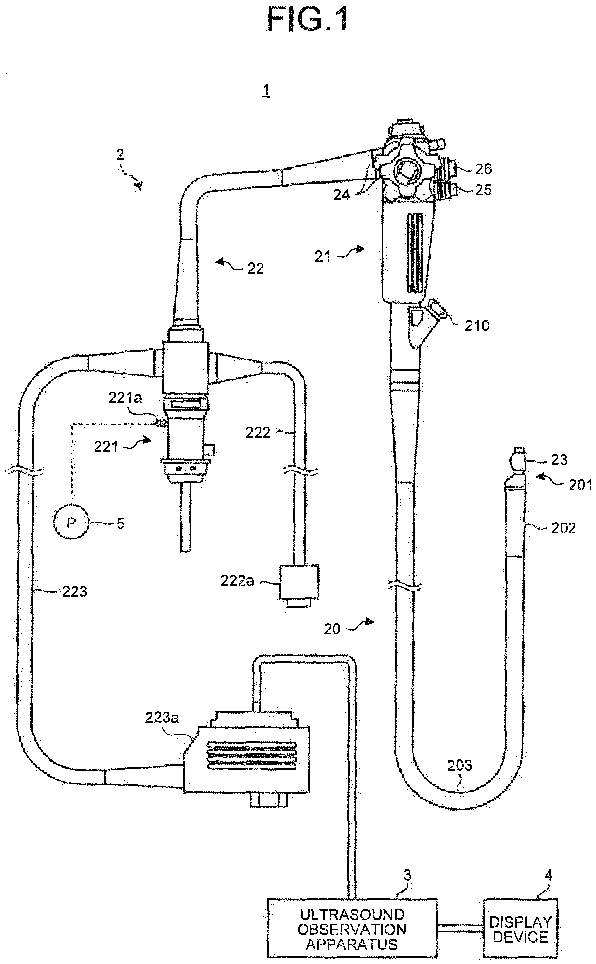

FIG. 1 is a schematic diagram illustrating an ultrasound diagnosis system according to a first embodiment of the present invention. An ultrasound diagnosis system 1 illustrated in the diagram includes an ultrasound endoscope 2, an ultrasound observation apparatus 3, a display device 4, and a suction pump 5. The ultrasound endoscope 2 transmits ultrasound to a subject as an observation target and receives the ultrasound reflected from the subject. The ultrasound observation apparatus 3 generates an ultrasound image based on an ultrasound signal obtained by the ultrasound endoscope 2. The display device 4 displays the ultrasound image generated by the ultrasound observation apparatus 3. The suction pump 5 generates a suction force to perform suction of body fluids and suction of a wall surface inside the subject.

The ultrasound endoscope 2 includes an insertion unit 20, an operating unit 21, and a universal cord 22. The insertion unit 20 has a thin and elongated shape and is inserted into the subject. The operating unit 21 is provided at a proximal end of the insertion unit 20. The universal cord 22 extends from a side portion of the operating unit 21.

A connector 221 is arranged at a proximal end portion of the universal cord 22. The connector 221 is connected to a light source apparatus (not illustrated). Cables 222 and 223 extend from the connector 221. The cable 222 is connected to a camera control unit (not illustrated) via a connector 222a. The cable 223 is removably connected to the ultrasound observation apparatus 3 via a connector 223a. The ultrasound endoscope 2 is connected with the ultrasound observation apparatus 3 via the connector 223a. The ultrasound endoscope 2 is further connected with the display device 4 via the ultrasound observation apparatus 3.

The connector 221 includes a suction cap 221a as a suction port of a suction channel (passage) extending to the distal end of a distal end portion 201. The suction cap 221a is connectable to the suction pump 5. The ultrasound endoscope 2 can suck the body fluids and suck a wall surface inside the subject, via the suction pump 5.

Main portions of the insertion unit 20 include, in the order from the distal end side, a distal end rigid portion (hereinafter, referred to as a distal end portion) 201, a bending portion 202, and a flexible tube portion 203. The bending portion 202 is located at a rear end of the distal end portion 201. The flexible tube portion 203 is long and flexible portion having a small diameter, located at a rear end of the bending portion 202, and extends to the operating unit 21.

An ultrasound transducer 23 is arranged on the distal end side of the distal end portion 201. Located on the proximal end side of the ultrasound transducer 23, the distal end portion 201 includes an illumination lens forming an illumination optical system, an observing lens in an observing optical system (both lenses not illustrated), and a forceps port, namely, a distal end opening combining a treatment tool insertion passage outlet and a suction port.

The operating unit 21 includes an angle knob 24, an air/water feeding button 25, a suction button 26 (suction force adjustment apparatus), and a treatment tool insertion port 210. The angle knob 24 controls bending of the bending portion 202 in a desired direction. The air/water feeding button 25 performs air/water feeding operation. The suction button 26 is located at a portion of the suction channel (passage) and performs suction operation. The treatment tool insertion port 210 is an entrance of treatment tools to be introduced into the body.

The ultrasound transducer 23 may be any of a convex transducer, a linear transducer, and a radial transducer. The ultrasound endoscope 2 may cause the ultrasound transducer 23 to perform mechanical scan, or may provide, as the ultrasound transducer 23, a plurality of elements in an array, and may cause the ultrasound transducer to perform electronic scan by electronically switching elements related to transmission/reception or imposing delay onto transmission/reception of each of elements.

The ultrasound endoscope 2 typically includes imaging optics and imaging elements. The ultrasound endoscope 2 can be inserted into gastrointestinal tracts (esophagus, stomach, duodenum, and large intestine) or respiratory organs (trachea, bronchus) of the subject and can image gastrointestinal tract, respiratory organs, and their surrounding organs (pancreas, gall bladder, bile duct, biliary tract, lymph nodes, mediastinal organs, blood vessels, or the like). The ultrasound endoscope 2 includes a light guide that guides illumination light emitted to the subject at the time of imaging. The light guide is configured such that a distal end portion thereof reaches a distal end of an insertion unit of the ultrasound endoscope 2 into the subject, while a proximal end thereof is connected to a light source device that generates illumination light.

The ultrasound observation apparatus 3 controls the entire ultrasound diagnosis system 1. The ultrasound observation apparatus 3 includes a CPU having calculation/control functions, various calculation circuits, or the like. The ultrasound observation apparatus 3 integrally controls the ultrasound observation apparatus 3 by reading information stored in a storage unit (not illustrated) and executing various types of calculation processing related to an operation method of the ultrasound observation apparatus 3.

The ultrasound observation apparatus 3 is electrically connected with the ultrasound endoscope 2, transmits a transmission signal (pulse signal) formed with a high-voltage pulse to the ultrasound transducer 23 on the basis of a predetermined waveform and transmission timing, and together with this, receives an echo signal, namely, an electrical reception signal, from the ultrasound transducer 23, and generates image data including an ultrasound image on the basis of the received echo signal. The ultrasound observation apparatus 3 generates, as an exemplary ultrasound image, a B-mode image, that is, a gray-scale image in which values of R (red), G (green) and B (blue), which are variables when the RGB color system is employed as a color space, match with one another.

Moreover, on the basis of a difference between a signal obtained when the ultrasound endoscope 2 is pressed against an observation target and a signal obtained when the ultrasound endoscope 2 is not pressed against the observation target, for example, the ultrasound observation apparatus 3 obtains information related to stiffness of the observation target in a set region, superposes color information corresponding to the stiffness, onto the B-mode image, and generates an elastographic image.

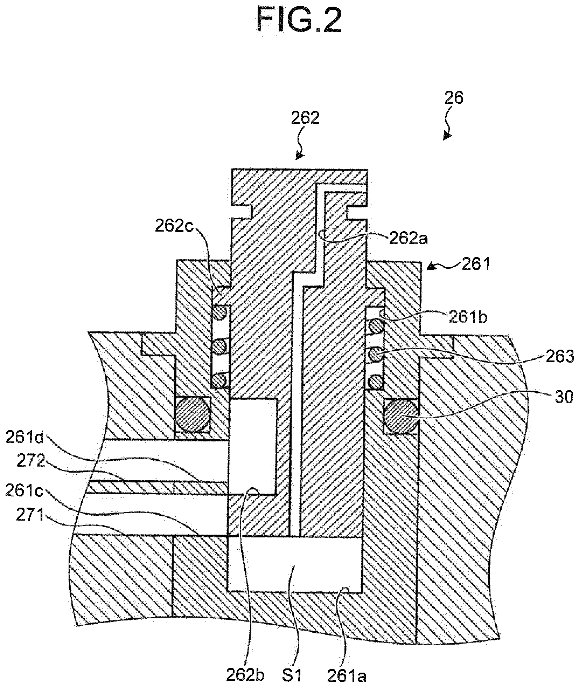

Subsequently, the suction button 26 that performs suction operation will be described with reference to the drawings. FIG. 2 is a schematic diagram illustrating a configuration of the suction button included in the ultrasound endoscope in the ultrasound diagnosis system according to the first embodiment, illustrating a state where pressing force is not applied to a piston unit 262. The suction button 26 includes a cylinder unit 261, the piston unit 262, and a spring unit 263.

The cylinder unit 261 has a cylindrical shape bottomed on one side. The cylinder unit 261 includes a housing unit 261a, a locking unit 261b, a first communicating portion 261c, and a second communicating portion 261d. The housing unit 261a forms a columnar hollow space capable of forward/backward movably housing the piston unit 262. The locking unit 261b is formed by enlarging a portion of the housing unit 261a and can be locked with the piston unit 262. The first communicating portion 261c allows communication between the inside/outside of the housing unit 261a and is connected to a first suction tube 271. The second communicating portion 261d allows communication between the inside/outside of the housing unit 261a and is connected with a second suction tube 272. In the first embodiment, the first suction tube 271 forms a portion of a suction channel (passage), one end communicating with the first communicating portion 261c, the other end connecting with the suction pump 5. The second suction tube 272 forms a portion of a suction channel (passage), one end communicating with the second communicating portion 261d, the other end communicating with the outside via the distal end portion 201.

The cylinder unit 261 is attached onto the operating unit 21 by fitting, for example. At this time, an O-ring 30 is provided between the operating unit 21 and the cylinder unit 261. With the O-ring 30, sealability and slip prevention between the operating unit 21 and the cylinder unit 261 can be maintained. The cylinder unit 261 may be fixed with a screw or adhesive such as sealant, other than fitting.

The piston unit 262 has a substantially columnar shape extending in accordance with the shape of the cross-section corresponding to the housing unit 261a. The piston unit 262 includes a communication hole 262a formed to allow communication between one end portion in the longitudinal direction (forward/backward direction with respect to the cylinder unit 261) and the other end portion. The communication hole 262a allows communication between a hollow space S1 (hollow portion) formed by the cylinder unit 261 (the housing unit 261a) and by the piston unit 262, and the outside. In the first embodiment, the suction button 26 except the communication hole 262a configures a suction force change unit.

The piston unit 262 includes a recess 262b and a flange unit 262c. The recess 262b is formed by cutting a portion of a side surface. The flange unit 262c is a portion protruding in a direction orthogonal to the longitudinal direction of the piston unit 262. The recess 262b includes an opening that allows communication between the first communicating portion 261c and the second communicating portion 261d.

The flange unit 262c is housed in the locking unit 261b, its moving range being regulated by the locking unit 261b. In addition, the spring unit 263 is provided at a portion between the flange unit 262c and the locking unit 261b, at a distal end side in the insertion direction of the piston unit 262.

The spring unit 263 is formed with a coil spring, for example. As described above, the spring unit 263 is provided between the flange unit 262c and the locking unit 261b, being arranged to be capable of biasing in the direction of causing the piston unit 262 to move out from the cylinder unit 261. Accordingly, in a case where force such as pressing force (force except gravity, or the like) is not applied, the piston unit 262 is maintained, by the biasing force of the spring unit 263, in a state where the flange unit 262c is locked with an outer end portion of the locking unit 261b, namely, a state where the piston unit 262 protrudes from the cylinder unit 261.

In a state where the piston unit 262 protrudes from the cylinder unit 261 (refer to FIG. 2), while the piston unit 262 includes an opening of the second communicating portion 261d within a region formed by the opening of the recess 262b and allows communication between the recess 262b and the second communicating portion 261d, the piston unit 262 closes the first communicating portion 261c by the side wall of the piston unit 262. Accordingly, the first suction tube 271 and the second suction tube 272 do not communicate with each other, causing no suction force by the suction pump 5, on the distal end portion 201.

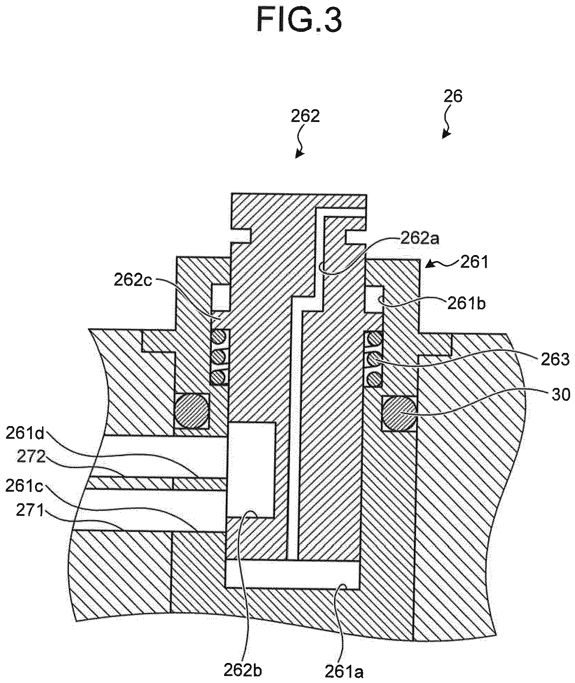

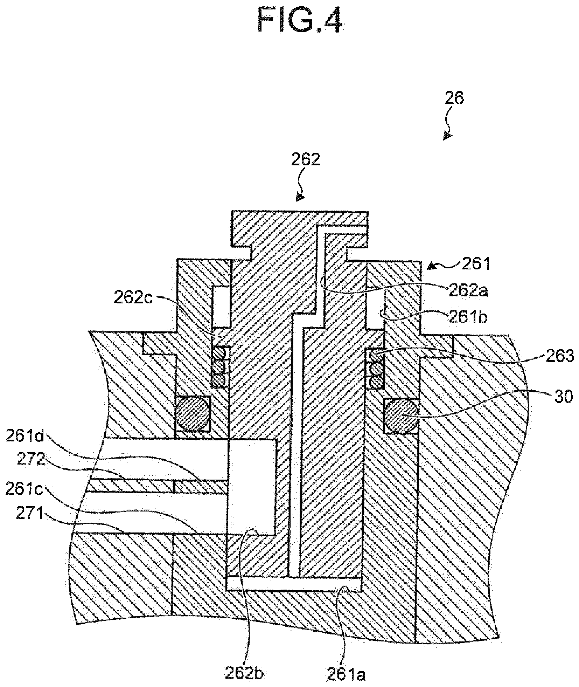

FIGS. 3 and 4 are schematic diagrams illustrating a configuration of the suction button included in the ultrasound endoscope in the ultrasound diagnosis system according to the first embodiment, illustrating a state where pressing force is applied to the piston unit 262. Pressing the piston unit 262 causes the piston unit 262 to be inserted into the cylinder unit 261. At this time, the gas inside the hollow space S1 illustrated in FIG. 2 is discharged to the outside via the communication hole 262a. When insertion operation is continued and the opening of the recess 262b comes to include the first communicating portion 261c and the second communicating portion 261d (facing each of the communicating portions) (refer to FIG. 3), a communication state is established between the first communicating portion 261c and the second communicating portion 261d, and as a result, communication is established between the first suction tube 271 and the second suction tube 272. Thereafter, pressing the piston unit 262 would allow the whole region of the opening of the first communicating portion 261c, which has been closed by the recess 262b, to be in an open state (refer to FIG. 4). Movement of the piston unit 262 opens the opening of the first communicating portion 261c, and this generates suction force by the suction pump 5 on the distal end portion 201, and thus, an internal wall surface of the subject, or the like, is sucked via the distal end portion 201.

In the first embodiment, in a case where the pressing force is not applied to the piston unit 262 (in a case where the piston unit 262 is not pressed into the cylinder unit 261), the communication between the first suction tube 271 and the second suction tube 272 is not established. Accordingly, the distal end portion 201 comes into either a contact or separate state with respect to the internal wall surface of the subject. In contrast, the pressing force is applied to the piston unit 262 (in a case where the piston unit 262 is pressed into the cylinder unit 261), communication between the first suction tube 271 and the second suction tube 272 is established via the recess 262b. Accordingly, the distal end portion 201 comes into a press-contact state while sucking the internal wall surface of the subject.

In addition, by adjusting the discharge of gas inside the hollow space S1 to the outside by decreasing the cross-section area of a portion orthogonal to the communication direction, on the communication hole 262a, it is possible to generate force that opposes insertion of the piston unit 262, and thus, to suppress an increase in the insertion speed. In addition, in a case where the pressing force applied to the piston unit 262 is released, the flowrate of the gas flowing into the hollow space S1 is limited by the communication hole 262a, causing the piston unit 262 to gradually retreat from the housing unit 261a. In this manner, the communication hole 262a functions as a damper unit to suppress the forward/backward movement speed of the piston unit 262. Due to this function as the damper unit, the piston unit 262 performs forward/backward operation in a substantially fixed speed with respect to the cylinder unit.

When the piston unit 262 performs gradual forward/backward operation from the housing unit 261a by the above-described damper unit, the opening area of the opening of the first suction tube 271 formed by the recess 262b of the piston unit 262 also gradually increases. Accordingly, also the suction force acting on the second suction tube 272 gradually increases in accordance with the opening state of the opening of the first suction tube 271.

As described above, according to the presence or absence of the pressing force applied to the piston unit 262, it is possible to easily switch the contact state of the distal end portion 201 toward the subject, and the press-contact state of the distal end portion 201 toward the subject, induced by suction by the distal end portion 201. Moreover, since the communication hole 262a controls the forward/backward movement speed of the piston unit 262, it is possible to periodically switch the contact state at a more fixed interval, toward the subject. By periodically switching the contact state, it is possible to stably obtain an echo signal in each of the states, and thus to generate a good elastographic image.

According to the above-described first embodiment, with the piston unit 262 configured to be inserted into the cylinder unit 261 and to retreat from the cylinder unit 261 by the biasing force of the spring unit 263, the communication hole 262a is provided to allow communication between the housing unit 261a and the outside so as to suppress the forward/backward movement speed of the piston unit 262. With this configuration, it is possible to stably obtain a good elastographic image with a simple configuration without increasing the size of the distal end portion.

The above-described first embodiment assumes that the locking unit 261b is provided at portions surrounding the housing unit 261a. Alternatively, however, in order to prevent rotation of the piston unit 262 around the longitudinal-axis, with respect to the cylinder unit 261, the locking unit 261b may have a groove-like shape extending in the forward/backward direction of the piston unit 262, may house a plurality of flange units formed corresponding to the groove-like shape, and together with this, each of the locking units 261b may house a spring unit (e.g., coil spring that can be housed in each of the grooves) and perform biasing.

### (Modification of First Embodiment)

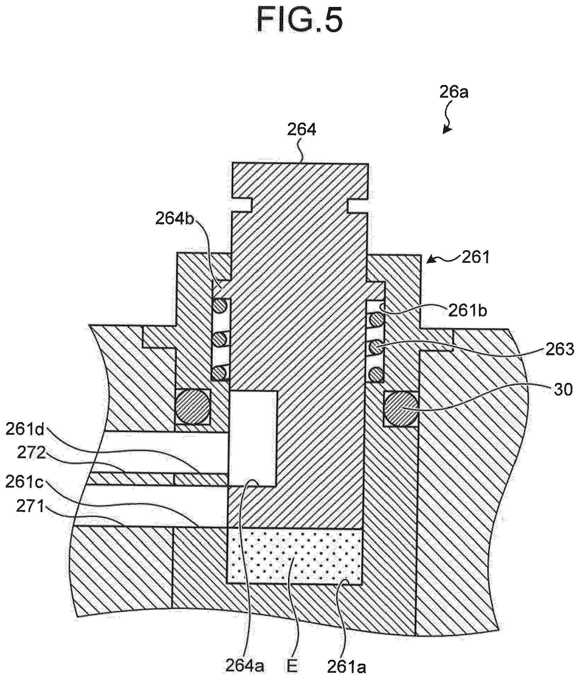

Next, a modification of the first embodiment of the present invention will be described. FIG. 5 is a schematic diagram illustrating a configuration of a suction button included in an ultrasound endoscope in an ultrasound diagnosis system according to a first modification of the first embodiment, illustrating a state where pressing force is not applied to a piston unit 264. The same reference signs are used to designate the same elements as those of the above-described embodiment. The above-described first embodiment assumes that the piston unit 262 includes the communication hole 262a as a damper unit, In the modification, however, the piston unit 264 includes an elastic member E as a damper unit.

A suction button 26a according to the modification includes the cylinder unit 261, the piston unit 264, the spring unit 263, and the elastic member E. The piston unit 264 includes a recess 264a and a flange unit 264b. The recess 264a is formed by cutting a portion of a side surface. The flange unit 264b is a portion protruding in a direction orthogonal to the longitudinal direction of the piston unit 264. The recess 264a includes an opening that allows communication between the first communicating portion 261c and the second communicating portion 261d.

The flange unit 264b is housed in the locking unit 261b, its moving range being regulated by the locking unit 261b. In addition, the spring unit 263 is provided at a portion between the flange unit 264b and the locking unit 261b, at a distal end side in the insertion direction of the piston unit 264.

In a state where the piston unit 264 protrudes from the cylinder unit 261, the piston unit 264, while the piston unit 264 includes an opening of the second communicating portion 261d within a region formed by the opening of the recess 264a and allows communication between the recess 264a and the second communicating portion 261d, the piston unit 264 closes the first communicating portion 261c by the side wall of the piston unit 264. Accordingly, the first suction tube 271 and the second suction tube 272 do not communicate with each other, causing no suction force by the suction pump 5, on the distal end portion 201.

The elastic member E is arranged at a hollow space formed by the cylinder unit 261 (housing unit 261a) and the piston unit 264, and fills the hollow space. The elastic member E is formed, for example, with elastomer having elastomeric quality, and resign having a predetermined elastic force.

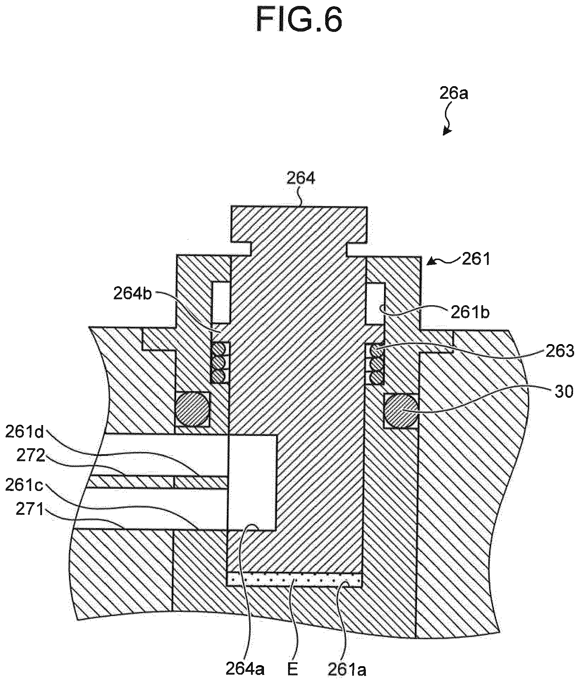

FIG. 6 is a schematic diagram illustrating a configuration of the suction button included in the ultrasound endoscope in the ultrasound diagnosis system according to the modification, illustrating a state where pressing force is applied to the piston unit 264. Pressing the piston unit 264 causes the piston unit 264 to be inserted into the cylinder unit 261 as illustrated in FIG. 6. When insertion operation is continued and the opening of the recess 264a comes to include the first communicating portion 261c and the second communicating portion 261d (facing each of the communicating portions), a communication state is established between the first communicating portion 261c and the second communicating portion 261d, and as a result, communication is established between the first suction tube 271 and the second suction tube 272. As a result, a suction force by the suction pump 5 is generated on the distal end portion 201, and thus, an internal wall surface of the subject, or the like, is sucked via the distal end portion 201.

In the modification, in a case where the pressing force is not applied to the piston unit 264 (in a case where the piston unit 264 is not pressed into the cylinder unit 261), the communication between the first suction tube 271 and the second suction tube 272 is not established. Accordingly, the distal end portion 201 comes into either a contact or separate state with respect to the internal wall surface of the subject. In contrast, the pressing force is applied to the piston unit 264 (in a case where the piston unit 264 is pressed into the cylinder unit 261), communication between the first suction tube 271 and the second suction tube 272 is established via the recess 264a. Accordingly, the distal end portion 201 comes in a press-contact state while sucking the internal wall surface of the subject.

Moreover, the elastic member E is arranged between the cylinder unit 261 (housing unit 261a) and the piston unit 264, making it possible to suppress an increase in the insertion speed of the piston unit 264 by the elastic force of the elastic member E. In addition, in a case where the pressing force on the piston unit 264 is released, a force (restoring force) acting on the elastic member E to return to an original shape causes the piston unit 264 to gradually retreat from the housing unit 261a.

As described above, according to the presence or absence of the pressing force applied to the piston unit 264, it is possible to easily switch the contact state of the distal end portion 201 toward the subject, and the press-contact state of the distal end portion 201 toward the subject, induced by suction by the distal end portion 201. Moreover, since the elastic member E controls the forward/backward movement speed of the piston unit 264, it is possible to periodically switch the contact state at a more fixed interval, toward the subject. By periodically switching the contact state, it is possible to stably obtain an echo signal in each of the states, and thus to generate a good and stable elastographic image.

As described above, according to the modification, with the piston unit 264 configured to be inserted into the cylinder unit 261 and to retreat from the cylinder unit 261 by the biasing force of the spring unit 263, the elastic member E is arranged at a hollow space formed by the cylinder unit 261 (housing unit 261a) and the piston unit 264 so as to suppress the forward/backward movement speed of the piston unit 264 by the elastic force of the elastic member E. With this, it is possible to stably obtain a good elastographic image with a simple configuration without increasing the size of the distal end portion.

### (Second Embodiment)

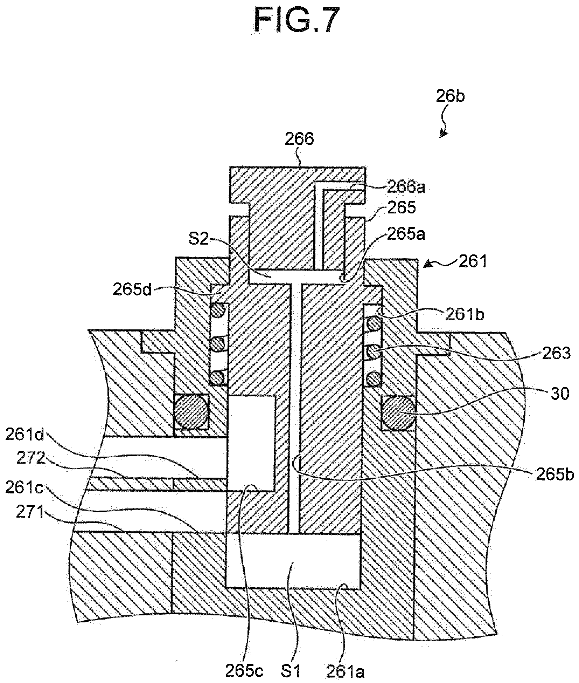

Next, a second embodiment of the present invention will be described. FIG. 7 is a schematic diagram illustrating a configuration of a suction button included in an ultrasound endoscope in an ultrasound diagnosis system according to the second embodiment, illustrating a state where pressing force is not applied to a piston unit 265. The same reference signs are used to designate the same elements as those of the above-described embodiment. The above-described first embodiment assumes that the piston unit 262 includes the communication hole 262a as a damper unit. In the second embodiment, however, the piston unit 265 includes a diaphragm mechanism to change the cycle of reciprocation of the piston unit 265.

A suction button 26b according to the second embodiment includes the cylinder unit 261, the piston unit 265, and the spring unit 263. The piston unit 265 has a substantially columnar shape. The piston unit 265 includes an opening portion 265a and a first communication hole 265b. The opening portion 265a is provided at an insertion-side rear end portion toward the cylinder unit 261. The first communication hole 265b allows communication between the opening portion 265a and an insertion-side distal end portion toward the cylinder unit 261. The piston unit 265 includes a recess 265c and a flange unit 265d. The recess 265c is formed by cutting a portion of a side surface. The flange unit 265d is a portion protruding in a direction orthogonal to the longitudinal direction of the piston unit 265.

The opening portion 265a can be screwed to an adjustment member 266 as an adjustment mechanism for changing the cycle of reciprocation of the piston unit 265. The adjustment member 266 includes a second communication hole 266a that allows communication between one end side and the other end side, of the direction of inserting into the opening portion 265a by screwing.

The recess 265c includes an opening that allows communication between the first communicating portion 261c and the second communicating portion 261d.

The flange unit 265d is housed in the locking unit 261b, its moving range being regulated by the locking unit 261b. In addition, the spring unit 263 is provided at a portion between the flange unit 265d and the locking unit 261b, at a distal end side in the insertion direction of the piston unit 265.

In a state where the piston unit 265 protrudes from the cylinder unit 261, while the piston unit 265 is configured such that the opening of the recess 265c is allowed to communicate with the second communicating portion 261d, the first communicating portion 261c is blocked by the side wall of the piston unit 265. Accordingly, the first suction tube 271 and the second suction tube 272 do not communicate with each other, causing no suction force by the suction pump 5, on the distal end portion 201.

In addition, by adjusting the discharge of gas inside the hollow space S1 to the outside by the communication hole to be formed by the first communication hole 265b and the second communication hole 266a, it is possible to generate force that opposes insertion of the piston unit 265, and thus, to suppress an increase in the insertion speed. In addition, in a case where the pressing force on the piston unit 265 is released, the flowrate of the gas flowing into the hollow space S1 is limited by the first communication hole 265b and the second communication hole 266a, causing the piston unit 265 to gradually retreat from the housing unit 261a. In this manner, each of the first communication hole 265b and the second communication hole 266a functions as a damper unit to suppress the forward/backward movement speed of the piston unit 265.

By changing the volume of a hollow space S2 to be formed between the adjustment member 266 and the opening portion 265a by rotating the adjustment member 266, it is possible to change the discharge of gas inside the hollow space S1 to the outside. With this configuration, it is possible to adjust the force that opposes insertion of the piston unit 265, change the insertion speed of the piston unit 265, and change the retreat speed of the piston unit 265 from the housing unit 261a.

As described above, according to the presence or absence of the pressing force applied to the piston unit 265, it is possible to easily switch the contact state of the distal end portion 201 toward the subject, and the press-contact state of the distal end portion 201 toward the subject, induced by suction by the distal end portion 201. Moreover, since the damper unit controls the forward/backward movement speed of the piston unit 265, it is possible to periodically switch the contact state toward the subject at a more fixed interval. By periodically switching the contact state, it is possible to stably obtain an echo signal in each of the states, and thus to generate a good and stable elastographic image.

As described above, according to the second embodiment, with the piston unit 265 configured so as to be inserted into the cylinder unit 261 and to retreat from the cylinder unit 261 by the biasing force of the spring unit 263, and with the adjustment member 266, the damper unit is formed to allow communication between the housing unit 261a and the outside so as to suppress the forward/backward movement speed of the piston unit 265. With this, it is possible to stably obtain a good elastographic image with a simple configuration without increasing the size of the distal end portion.

In addition, according to the above-described second embodiment, the discharge of gas inside the hollow space S1 to the outside is changed by changing the volume of the hollow space S2 formed between the adjustment member 266 and the opening portion 265a by rotating the adjustment member 266. With this configuration, it is possible to adjust the force that opposes insertion of the piston unit 265, change the insertion speed of the piston unit 265, and change the retreat speed of the piston unit 265 from the housing unit 261a.

The above-described second embodiment assumes that the piston unit 265 and the adjustment member 266 are screwed with each other. Alternatively, however, the adjustment member 266 may be press-fitted into the piston unit 265. In this case, it would be allowable to configure such that a protrusion (or recess) is provided on an inner peripheral surface of the opening portion 265a and that a recess (or protrusion) is provided on an outer peripheral surface of the adjustment member 266, and that gradual positioning of the adjustment member 266 is performed with respect to the opening portion 265a.

### (Third Embodiment)

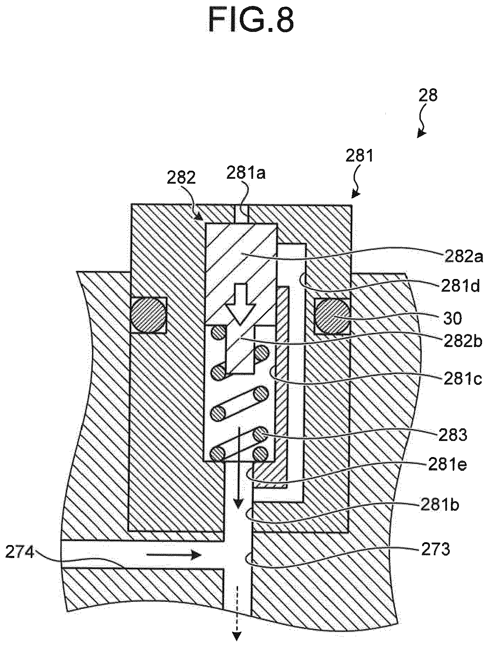

Next, a third embodiment of the present invention will be described. FIG. 8 is a schematic diagram illustrating a configuration of a suction button included in an ultrasound endoscope in an ultrasound diagnosis system according to the third embodiment of the present invention. The same reference signs are used to designate the same elements as those of the above-described embodiments. The above-described first embodiment assumes that suction operation is performed by applying pressing force to the piston unit 262. In the third embodiment, however, suction operation by the distal end portion 201 is performed by using the suction force by the suction pump 5.

A suction button 28 according to the third embodiment includes a cylinder unit 281, a piston unit 282, and a spring unit 283.

The cylinder unit 281 has a hollow cylindrical shape (tubular shape bottomed on both sides), having opening portions 281a and 281b formed on the top and bottom surfaces, respectively. The cylinder unit 281 includes a housing unit 281c, a bypass tube (bypass tube passage) 281d, and a communicating portion 281e. The housing unit 281c communicates with the opening portions 281a and 281b at its both ends, respectively, and forms a columnar hollow space (hollow portion) that can movably house the piston unit 282. The bypass tube 281d extends from a side wall on the opening portion 281a side of the housing unit 281c and communicates onto the opening portion 281b. The communicating portion 281e allows communication between the opening portion 281b and the housing unit 281c. In the third embodiment, a first suction tube 273 forms a portion of a suction channel (passage), one end communicating with the opening portion 281b, the other end connecting to the suction pump 5 (not illustrated). A second suction tube 274 forms a portion of a suction channel (passage), one end connecting to the first suction tube 273, the other end communicating with the outside via the distal end portion 201. In the third embodiment, the opening portion 281a allows communication between the outside and the housing unit 281c, and the opening portion 281b allows communication between the housing unit 281c and the first suction tube 273.

The cylinder unit 281 is attached onto the operating unit 21 by fitting, for example. At this time, the O-ring 30 is provided between the operating unit 21 and the cylinder unit 281. With the O-ring 30, sealability and slip prevention between the operating unit 21 and the cylinder unit 281 can be maintained.

The piston unit 282 is slidably disposed inside the housing unit 281c. The piston unit 282 includes a sliding unit 282a and an extending portion 282b. The sliding unit 282a slides with respect to the housing unit 281c. The extending portion 282b having a cylindrical shape corresponds to one end of the sliding unit 282a and extends from a surface facing the opening portion 281b. The spring unit 283 is provided between an end portion on the extending portion 282b side of the piston unit 282 and an end portion on the opening portion 281b side of the housing unit 281c.

The spring unit 283 is formed with a coil spring, for example. As described above, the spring unit 283 is provided between the extending portion 282b and the housing unit 281c, being arranged to be capable of biasing in the direction of causing the piston unit 282 to move out from the cylinder unit 281 (direction toward the opening portion 281a). The spring unit 283 may be configured to be press-fitted into the extending portion 282b, or to enclose the extending portion 282b without being press-fitted into the extending portion 282b. It would be sufficient that the extending portion 282b can lock the spring unit 283 to prevent the spring unit 283 from tilting.

In a state where the piston unit 282 is positioned on the opening portion 281a side of the cylinder unit 281, the piston unit 282 closes the opening of the bypass tube 281d. Accordingly, the first suction tube 273 does not communicate with the outside, causing the suction force by the suction pump 5 to act on the housing unit 281c, at the distal end portion 201. In a case where the first suction tube 273 does not communicate with the outside, the suction force by the suction pump 5 acts on the housing unit 281c and on the second suction tube 274. In other words, the suction force acts on the second suction tube 274, whereby the internal wall surface of the subject or the like is sucked via the distal end portion 201.

When the gas within the internal space of the housing unit 281c is sucked by the suction pump 5 via the communicating portion 281e and the opening portion 281b, the internal pressure of the housing unit 281c is lowered, whereby the piston unit 282 moves in the direction to decrease the volume of the housing unit 281c (direction toward the opening portion 281b). When the piston unit 282 moves toward the opening portion 281b side, the bypass tube 281d comes in communication with the outside via the opening portion 281a and the housing unit 281c.

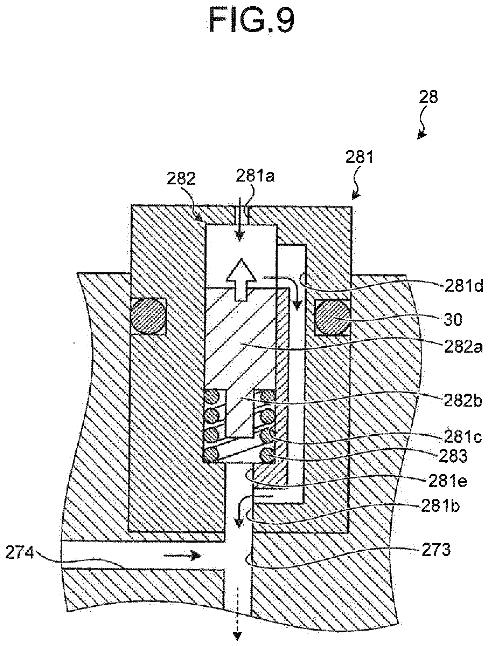

FIG. 9 is a schematic diagram illustrating a configuration of a suction button included in the ultrasound endoscope in the ultrasound diagnosis system according to the third embodiment, illustrating a state where the piston unit 282 has moved to the opening portion 281b side. As illustrated in FIG. 9, when the piston unit 282 moves to the opening portion 281b side by the suction force by the suction pump 5, the bypass tube 281d comes in communication with the outside, and then, the suction force by the suction pump 5 begins to act on the second suction tube 274 and together with this, act as suction force to suck the outside gas. With this action, the suction force by the distal end portion 201 via the second suction tube 274 decreases compared with a case where the bypass tube 281d does not communicate with the outside (refer to FIG. 8).

In the third embodiment, in a case where the first suction tube 273 does not communicate with the outside (in a case where the piston unit 282 closes the bypass tube 281d), the suction force by the second suction tube 274 is relatively great, and thus, the distal end portion 201 comes in a press-contact state while sucking the internal wall surface of the subject. In contrast, in a case where the first suction tube 273 is in a communication state with the outside (case where the piston unit 282 opens the bypass tube 281d), suction force by the second suction tube 274 is relatively small. Accordingly, the distal end portion 201 comes into either a contact or separate state with respect to the internal wall surface of the subject.

When the first suction tube 273 comes in communication with the outside, the housing unit 281c that is in a low-pressure state gradually returns to a state of normal pressure. Accordingly, the piston unit 282 gradually moves again to the opening portion 281a side by increased pressure and the biasing force of the spring unit 283. Along with this movement of the piston unit 282, the bypass tube 281d that has been in an open state with the outside is blocked again. With this change, due to the suction operation of the suction pump 5, the piston unit 282 reciprocates within the housing unit 281c, while the suction state by the distal end portion 201 periodically changes. At this time, similarly to the above-described communication hole 262a, the bypass tube 281d can adjust the discharge to the outside, by decreasing the cross-section orthogonal to the communication direction. With this adjustment, the bypass tube 281d generates force that opposes the movement of the piston unit 282, and thus, functions as a damper unit to suppress the forward/backward movement speed of the piston unit 282. Note that the diameter of the bypass tube 281d is preferably smaller than the diameter of the first suction tube 273. In the third embodiment, the suction button 28 except the bypass tube 281d configures a suction force change unit.

According to the above-described third embodiment, the open state and closed state of the bypass tube 281d toward the outside is changed by the piston unit 282 that is moved by the internal pressure of the housing unit 281c, under the suction operation by the suction pump 5, and together with this, the forward/backward movement speed of the piston unit 282 is suppressed by the bypass tube 281d. With this configuration, it is possible to stably obtain a good elastographic image with a simple configuration without increasing the size of the distal end portion.

### (Fourth Embodiment)

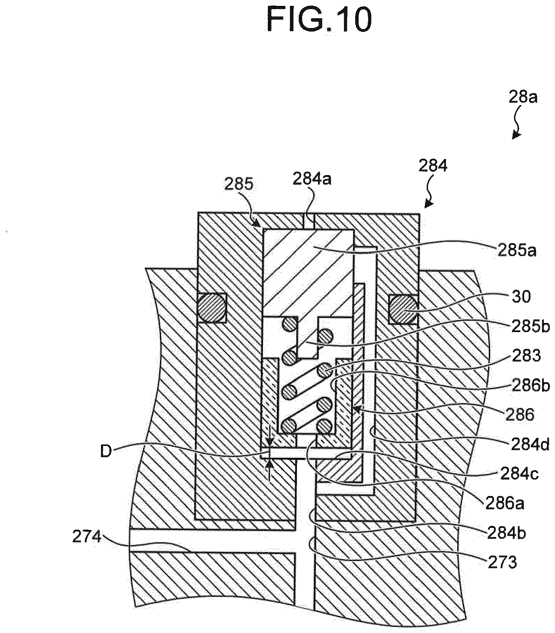

Next, a fourth embodiment of the present invention will be described. FIG. 10 is a schematic diagram illustrating a configuration of a suction button included in an ultrasound endoscope in an ultrasound diagnosis system according to the fourth embodiment of the present invention. The same reference signs are used to designate the same elements as those of the above-described embodiments. While the above-described third embodiment assumes that suction operation is performed by the distal end portion 201 using the suction force by the suction pump 5, the fourth embodiment includes a diaphragm mechanism to change the cycle of reciprocation of a piston unit 285.

A suction button 28a according to the fourth embodiment includes a cylinder unit 284, the piston unit 285, and the spring unit 283.

The cylinder unit 284 has a hollow cylindrical shape, having opening portions 284a and 284b formed on the top and bottom surfaces, respectively. The cylinder unit 284 includes a housing unit 284c and a bypass tube 284d. The housing unit 284c communicates with the opening portions 284a and 284b at its both ends, respectively, and includes a columnar hollow space that can movably house the piston unit 285. The bypass tube 284d extends from a side wall on the opening portion 284a side of the housing unit 284c and communicates onto the opening portion 284b. In the fourth embodiment, the first suction tube 273 is formed such that one end communicates with the opening portion 284b, the other end connecting to the suction pump 5 (not illustrated). In the fourth embodiment, the opening portion 284a allows communication between the outside and the housing unit 284c, while the opening portion 284b allows communication between the housing unit 284c and the first suction tube 273.

As an adjustment mechanism for changing the cycle of reciprocation of the piston unit 285, an adjustment member is provided inside the housing unit 284c. The adjustment member 286 has a bottomed cylindrical shape, has an opening portion 286a on the bottom, and includes a holding unit 286b that can house and hold a portion of the piston unit 285 and the spring unit 283. The adjustment member 286 can be screwed, on its outer peripheral surface, to the inner peripheral surface of the housing unit 284c. When the adjustment member 286 rotates around the longitudinal-axis, the position of the adjustment member 286 relative to the housing unit 284c changes.

Similarly to the cylinder unit 281, the cylinder unit 284 is attached onto the operating unit 21 by fitting, for example. At this time, the O-ring 30 is provided between the operating unit 21 and the cylinder unit 284.

The piston unit 285 is slidably disposed inside the housing unit 284c. The piston unit 285 includes a sliding unit 285a and an extending portion 285b. The sliding unit 285a slides with respect to the housing unit 284c. The extending portion 285b having a cylindrical shape corresponds to one end of the sliding unit 285a and extends from a surface facing the opening portion 284b. The above-described spring unit 283 is provided between an end portion on the extending portion 285b side of the piston unit 285 and the bottom of the adjustment member 286.

In the fourth embodiment, in a case where the first suction tube 273 does not communicate with the outside (in a case where the piston unit 285 closes the bypass tube 284d), the suction force by the second suction tube 274 is relatively great, and thus, the distal end portion 201 comes in a press-contact state while sucking the internal wall surface of the subject. In contrast, in a case where the first suction tube 273 is in a communication state with the outside (case where the piston unit 285 opens the bypass tube 284d), the suction force by the second suction tube 274 is relatively small. Accordingly, the distal end portion 201 comes into either a contact or separate state with respect to the internal wall surface of the subject.

When the first suction tube 273 comes in communication with the outside, the housing unit 284c that is in a low-pressure state gradually returns to a state of normal pressure. Accordingly, the piston unit 285 gradually moves again to the opening portion 284a side by increased pressure and the biasing force of the spring unit 283. Along with this movement of the piston unit 285, the bypass tube 284d that has been in an open state with the outside returns to a closed state. With this change, due to the suction operation of the suction pump 5, the piston unit 285 reciprocates within the housing unit 284c, while the suction state by the distal end portion 201 periodically changes. At this time, similarly to the above-described bypass tube 281d, the bypass tube 284d can adjust the discharge to the outside, by decreasing the cross-section orthogonal to the communication direction. With this adjustment, the bypass tube 284d generates force that opposes the movement of the piston unit 285, and thus, functions as a damper unit to suppress the forward/backward movement speed of the piston unit 285.

By changing a distance D with respect to the housing unit 284c, specifically, the distance between the outer surface of the bottom of the adjustment member 286 and the bottom surface of the housing unit 284c by rotating the adjustment member 286, a reciprocating distance (reciprocating cycle) of the piston unit 285 with respect to the housing unit 284c changes, making it possible to change the speed of the reciprocation of the piston unit 285.

As described above, by using the piston unit 285 that moves by the internal pressure of the housing unit 284c under the suction operation by the suction pump 5, it is possible to easily switch the contact state of the distal end portion 201 toward the subject, and the press-contact state of the distal end portion 201 toward the subject, induced by suction by the distal end portion 201. Moreover, since the damper unit controls the forward/backward movement speed of the piston unit 285, it is possible to periodically switch the contact state at a more fixed interval, toward the subject. By periodically switching the contact state, it is possible to stably obtain an echo signal in each of the states, and thus to generate a good and stable elastographic image.

According to the above-described fourth embodiment, the open state and closed state of the bypass tube 284d toward the outside are changed by the piston unit 285 that is moved by the internal pressure of the housing unit 284c, under the suction operation by the suction pump 5, and together with this, the forward/backward movement speed of the piston unit 285 is suppressed by the bypass tube 284d. With this configuration, it is possible to stably obtain a good elastographic image with a simple configuration without increasing the size of the distal end portion.

Moreover, according to the above-described fourth embodiment, the speed of the reciprocation of the piston unit 285 is to be changed by changing the distance D to the housing unit 284c by rotating the adjustment member 286. Accordingly, it is possible to change the insertion speed of the piston unit 285 and the retreat speed of the piston unit 285 from the housing unit 284c.

The above-described first to fourth embodiments assume that the suction button is a button to operate the contact or press-contact state toward the internal wall surface of the body by the distal end portion 201. In addition to this, the suction button can also be used to suck body fluid, or the like, when the piston unit is removed. It would be also allowable to arrange the suction button to operate the contact or press-contact state toward the internal wall surface of the body by the distal end portion 201 separately from the suction button to perform operation for sucking the body fluid, or the like. In this case, it is allowable to configure such that the passage for suction of the body fluid, or the like, differs from the passage for suction for performing press-contact of the distal end portion 201 to the internal wall surface of the body.

### (Fifth Embodiment)

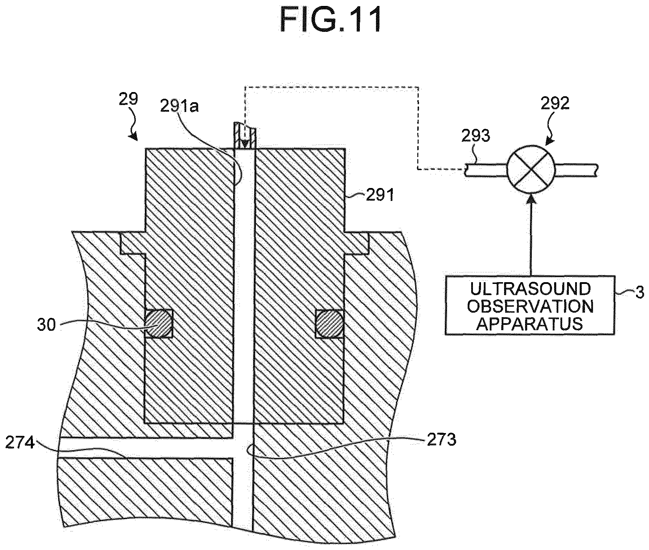

Next, a fifth embodiment of the present invention will be described. FIG. 11 is a schematic diagram illustrating a configuration of a suction force adjustment unit included in an ultrasound endoscope in an ultrasound diagnosis system according to the fifth embodiment of the present invention. The same reference signs are used to designate the same elements as those of the above-described embodiments. While the above-described first to fourth embodiments assume that periodical suction operation is performed by reciprocation of the piston unit, the fifth embodiment includes a suction force adjustment unit 29 instead of the above-described suction button. The suction force adjustment unit 29 controls (adjusts) a suction state of the second suction tube 274 by a solenoid valve 292.

The suction force adjustment unit 29 according to the fifth embodiment includes a main body unit 291, the solenoid valve 292, and piping 293. The main body unit 291 is attached onto the operating unit 21 by fitting. At this time, the O-ring 30 is provided between the operating unit 21 and the main body unit 291. The main body unit 291 includes a through-hole 291a. The through-hole 291a includes an opening on the external surface, while its other end is connected to the above-described first suction tube 273.

The solenoid valve 292 is provided on the piping 293 and opens and closes the valve by moving a plunger by a magnetic force of an electromagnet under the control of an external control apparatus such as the ultrasound observation apparatus 3. The piping 293 is configured such that its one end side is connected to the through-hole 291a and the other end side is connected to the outside. With this configuration, when the solenoid valve 292 is in a closed state, the inside of the piping 293 comes into a closed state, and the first suction tube 273 does not communicate with the outside. Therefore, the suction force by the suction pump 5 via the first suction tube 273 acts on the second suction tube 274. In contrast, when the solenoid valve 292 comes into an open state, the inside of the piping 293 is opened, and the first suction tube 273 comes into a communication state with the outside. Therefore the suction force by the suction pump 5 via the first suction tube 273 acts on the second suction tube 274, and together with this, acts as suction force that sucks external gas. The solenoid valve 292 may be opened by the control of the ultrasound observation apparatus 3, or may be controlled by a control apparatus other than the ultrasound observation apparatus 3, for example, an apparatus that controls open/close operation using an input button or a dial electrically connected to the solenoid valve 292.

Accordingly, when the solenoid valve 292 is in the closed state, the suction force by the suction pump 5 acts on the second suction tube 274, and thus, the internal wall surface inside the body of the subject is sucked via the distal end portion 201. In contrast, when the solenoid valve 292 comes into an open state, the suction force by the suction pump 5 acts on the second suction tube 274, and together with this, acts as the suction force to suck the external gas. Accordingly, the suction force by the second suction tube 274 becomes relatively small, and thus, the distal end portion 201 comes into either a contact or separate state with respect to the internal wall surface of the subject.

At this time, by decreasing the diameter of the piping 293, it is possible to adjust the discharge of gas to the outside. By decreasing the discharge, it is possible to gently change the suction force of the second suction tube 274. In this manner, the piping 293 functions as a damper unit that gently changes the suction force by the distal end portion 201. Note that the diameter of the piping 293 is preferably smaller than the diameter of the first suction tube 273. It is also allowable to provide the solenoid valve 292 and a portion of the piping 293, inside the main body unit 291.

According to the above-described fifth embodiment, the control by the solenoid valve 292 allows the open state and closed state of the first suction tube 273 to change toward the outside, and allows the suction force of the second suction tube 274 using the piping 293 to gently change, under the suction operation by the suction pump 5. With this configuration, it is possible to stably obtain a good elastographic image with a simple configuration without increasing the size of the distal end portion.

It is also allowable to connect the piping 293 to the bypass tube 281d using a combination of the above-described third and fifth embodiments.

Embodiments of the present invention have been described hereinabove, however, the present invention is not intended to be limited to the above-described embodiments. For example, while the above description assumes that the observation target is a living tissue, it is also applicable not only to the ultrasound endoscope but also to an endoscope that images inside the subject, and to an industrial endoscope for observing characteristics of a material. The endoscope according to the present invention is applicable both to external and internal portions of the body.

In this manner, the present invention may include various forms of embodiments without deviating from the technical ideas as defined in the appended claims of this invention.

### Industrial Applicability

As described above, a suction force adjustment apparatus for ultrasound examination, and an ultrasound endoscope according to the present invention are useful in stably obtaining a good elastographic image without increasing the size of the distal end portion, with a simple configuration.

### Reference Signs List

1 ULTRASOUND DIAGNOSIS SYSTEM
2 ULTRASOUND ENDOSCOPE
3 ULTRASOUND OBSERVATION APPARATUS
4 DISPLAY DEVICE
5 SUCTION PUMP
20 INSERTION UNIT
21 OPERATING UNIT
22 UNIVERSAL CORD
23 ULTRASOUND TRANSDUCER
24 ANGLE KNOB
25 AIR/WATER FEEDING BUTTON
26, 26a, 26b, 28, 28a SUCTION BUTTON
29 SUCTION FORCE ADJUSTMENT UNIT
30 O-RING
261, 281, 284 CYLINDER UNIT
262, 264, 265, 282, 285 PISTON UNIT
263, 283 SPRING UNIT
265a OPENING PORTION
265b FIRST COMMUNICATING HOLE
266, 286 ADJUSTMENT MEMBER
266a SECOND COMMUNICATING HOLE
281d, 284d BYPASS TUBE
E ELASTIC MEMBER

## Claims

1. A suction force adjustment apparatus used for ultrasound examination of an observation target by transmitting ultrasound to the observation target and receiving the ultrasound reflected from the observation target, the apparatus comprising:
a suction force change unit configured such that one end is configured to be connected to a suction pump and the other end is provided at a portion of a channel leading to a contact portion with the observation target, and configured to change suction force of the suction pump on the other end; and
a damper unit configured to suppress a change in the suction force by the suction force change unit.

2. The suction force adjustment apparatus for ultrasound examination according to claim 1, wherein
the suction force change unit comprises:
a cylinder unit having a cylindrical shape bottomed on one side, and having a first communicating portion for communicating with one end side of the channel and a second communicating portion for communicating with the other end side of the channel;
a piston unit slidable with respect to the cylinder unit, and configured to change a communication state between the first communicating portion and the second communicating portion according to a movement with respect to the cylinder unit; and
a spring unit configured to bias the piston unit toward a direction of causing the first communicating portion and the second communicating portion not to communicate with each other.

3. The suction force adjustment apparatus for ultrasound examination according to claim 2, wherein
the damper unit is provided in the piston unit, and is a communication hole that allows communication between outside and a hollow portion formed by the cylinder unit and the piston unit.

4. The suction force adjustment apparatus for ultrasound examination according to claim 2, wherein
the damper unit is an elastic member provided in a hollow portion formed by the cylinder unit and the piston unit.

5. The suction force adjustment apparatus for ultrasound examination according to any one of claims 2 to 4, further comprising an adjustment member configured to adjust volume of a hollow space formed by the communication hole.

6. The suction force adjustment apparatus for ultrasound examination according to claim 1, wherein
the suction force change unit comprises:
a cylinder unit having a hollow cylindrical shape, and having a first opening portion disposed on one end to communicate with outside and a second opening portion disposed on the other end to communicate with the channel;
a piston unit provided in a hollow portion of the cylinder unit and slidable between the one end and the other end of the cylinder unit; and
a spring unit configured to bias the piston unit from the other end of the cylinder unit toward the one end of the cylinder unit,
the damper unit is a bypass tube passage provided in the cylinder unit to allow communication between the one end and the other end of the cylinder unit, and
the piston unit is configured to slide with respect to the cylinder unit in accordance with one of pressure change due to the suction force and biasing force of the spring unit, thereby to open or close one end side of the bypass tube passage.

7. The suction force adjustment apparatus for ultrasound examination according to claim 6, further comprising an adjustment member configured to adjust a movement range of the piston unit with respect to the cylinder unit.

8. An ultrasound endoscope comprising:
an insertion unit;
an ultrasound transducer provided at a distal end of the insertion unit and configured to transmit ultrasound to an observation target and to receive the ultrasound reflected from the observation target;
a suction force change unit configured such that one end is configured to be connected to a suction pump and the other end is provided at a portion of a channel leading to the distal end of the insertion unit, and configured to change suction force of the suction pump on the other end; and
a damper unit configured to suppress a change in the suction force by the suction force change unit.

9. An ultrasound endoscope comprising:
an insertion unit;
an ultrasound transducer provided at a distal end of the insertion unit and configured to transmit ultrasound to an observation target and to receive the ultrasound reflected from the observation target; and
a suction force adjustment unit configured such that one end leads to a suction pump and the other end is connected to a portion of a channel leading to the distal end of the insertion unit, and configured to adjust suction force of the suction pump under control of an external control apparatus, wherein
the suction force adjustment unit comprises a damper unit configured to adjust a discharge of gas from the channel.
